# EUROPEAN PATENT SPECIFICATION

(11) **EP 2 227 261 B1**
(45) Date of publication and mention of the grant of the patent: **14.05.2014**
(21) Application number: 08705830.1
(22) Date of filing: 10.01.2008
(51) Int. Cl.: A61L 17/04, A61L 31/02, A61B 17/06

(54) **TUNGSTEN ALLOY SUTURE NEEDLES WITH SURFACE COLORATION**
NÄHNADELN AUS EINER WOLFRAM-LEGIERUNG MIT OBERFLÄCHENFÄRBUNG
AIGUILLES À SUTURE EN ALLIAGE DE TUNGSTÈNE AVEC UNE SURFACE COLORÉE

(43) Date of publication of application: 15.09.2010
(73) Proprietor: ETHICON, INC., Somerville, NJ 08876-1515 (US)
(72) Inventor: CICHOCKI, Frank R., Jr., Easton Pennsylvania 18045 (US)
(74) Representative: Kirsch, Susan Edith
(86) International application number: PCT/US2008/050736
(87) International publication number: WO 2009/088515

(56) References cited:
- EP-A- 0 948 934
- WO-A-2009/076610
- US-A- 5 415 707
- US-A1- 2007 233 217

## Description

### FIELD OF THE INVENTION

The present invention relates to suture needles, and in particular to tungsten alloy suture needles having a desirable combination of stiffness and strength, and a robust surface coloration. More specifically, the present invention relates to tungsten alloy suture needles that exhibit a blue, yellow or black surface coloration, and methods for imparting the surface coloration to the needle.

### BACKGROUND OF THE INVENTION

Suture needles are often colored to assist a surgeon to visibly distinguish the needle and visually locate the needle point in the surgical field in which the surgeon is performing a surgery. For example, if the surgical field is filled with fluid it may be difficult for the surgeon to see a silver colored needle against the fluid, or against tissue that is surrounded by fluid such as saline or blood. Additionally, certain surgeries, particularly coronary artery bypass surgery, necessarily involve the use of suture needles of small diameter, which are difficult to visually distinguish in the surgical field due to their small size. In response to the need to visibly distinguish the suture needle from the surgical field, stainless steel needles have been colored black using coloration processes that require toxic chrome bearing solutions. Special steps are taken in order to ensure that the toxic solutions are completely removed from the suture needle, and special handling and disposal procedures are required due to their toxic nature. Further, when utilizing suture needles having small diameters, it is desirable for such needles to have exceedingly high bending stiffness and strength. In particular, surgery of this type requires that the suture needle's path be closely controlled. If the needle flexes excessively as it enters the tissue or as it pierces the inner surface of e.g., a blood vessel before re-emerging, improper placement of the needle and serious trauma to the tissue and the patient can occur. In use, suture needles are subjected to substantial stressing forces, since the force used to drive the needle into and through tissue (e.g., a blood vessel and the like) needs to be sufficient to overcome frictional drag through the tissue. These forces resisting needle penetration are commonly exacerbated in patients undergoing cardiovascular surgery, who exhibit calcified or toughened tissue due to coronary artery disease. In these procedures, the suture needle must be able to pass through not only the blood vessel, but also any hard calcified tissue that may be located along the periphery of the blood vessel lumen. A compliant needle will deflect elastically during tissue penetration resulting in a loss of placement control. As such, it is preferable that the needle should have a relatively high bending stiffness, that is, a low tendency to flex and high tendency to retain its configuration when subjected to a deforming force. Hence, stiffness in bending is an essential property for the handling and performance of suture needles. A stiff needle resists elastic deflection and can thus be directed as intended to provide a high level of control.

USP 5,415,707 describes tungsten alloys having exceptionally high stiffness, which has been found to be useful in producing suture needles having small diameters, especially curved needles. Tungsten alloys derive their strength from their high dislocation density and the natural resistance to deformation that occurs via dislocation-dislocation interaction as a stress is applied. This exceptionally high stiffness of such tungsten alloys in wire and straight needle form has been found to be reproducible in curved tungsten alloy needles if the curved needle is heat treated at a temperature below the recrystallization temperature of the alloy. Curved needles subjected to this heat treatment exhibit desirable bend properties such as high bending strength and high bending stiffness.

The tungsten alloy needles described in USP 5,415,707 do not exhibit colors, but rather exhibit the metallic silver appearance intrinsic to most polished metal alloys. Hence there remains a need for tungsten alloy suture needles that exhibit robust surface coloration, particularly for surgical procedures that require small diameter needles.

US-A-2007233217 describes implantable metal electrodes formed from a tungsten alloy. A low polarization coating can be applied to the electrodes, e.g. of platinum black, thereby rendering the surface coloration black.

EP-A-0948934 describes a surgical needle having an uncoated puncture tip and the remaining or rear section being matt-finished or colored by chemical or electrolytic means or by a covering.

WO-A-2009076610, which forms part of the state of the art by virtue of Article 54(3) EPC, describes thermal treatment and coloring of suture needles.

### SUMMARY OF THE INVENTION

In a first aspect the present invention provides a method for making a suture needle comprising the steps of (1) forming needle blanks of a tungsten alloy comprising up to 30 weight percent of one or more metals selected from rhenium, osmium, tantalum and molybdenum, and the balance tungsten, into a suture needle; and (2) subjecting the suture needle to a DC or AC potential ranging from 1 to 40 VDC while submerged in an aqueous solution with a pH of 7 or less, to impart a yellow, blue or black surface coloration to the needle.

In a second aspect, the present invention provides a suture needle obtainable by the method of the invention.

### DESCRIPTION OF THE DRAWINGS

Figure 1 is a graph showing the bending performance of tungsten 25.75% rhenium suture needles as a function of heat treatment temperature for a duration of 0.5 hour.
Figure 2 is a graph comparing the bending performance of a 0.2mm (.008") diameter suture needle produced from a tungsten 26% rhenium alloy to an equivalent suture needle produced from a 4310 stainless steel alloy.
Figure 3 is a process diagram for the electrochemical treatment of tungsten alloy suture needles.

### DETAILED DESCRIPTION OF THE INVENTION

The suture needle of the present invention is formed from an alloy of tungsten. The tungsten alloy may comprise one or more metals selected from the group consisting of rhenium, osmium, tantalum, or molybdenum. Preferably, the alloy is a tungsten-rhenium alloy, and has no more than trace amounts of other elements present. The metal other than tungsten may be present in an amount up to 30 weight percent of the alloy, and more preferably is present in an amount ranging from 20 to 26 weight percent of the alloy.

Preferably, the suture needle has a diameter effective to permit satisfactory usage in fine surgery. Typically, the diameter will be less than about 1524 µm (60 mils), preferably less than about 381 µm (15 mils), down to about 25 µm (1 mil), and preferably about 36 µm to about 305 µm (about 1.4 to about 12 mils). It will be recognized that the suture needle may have a circular body cross-section, and that the needle may also be of a non-circular cross-sectional shape such as triangular; trapezoidal; rectangular; hexagonal; elliptical; or rectangular wherein the opposed shorter ends of the rectangle are rounded into semicircles. By "diameter" herein is meant the square root of (4A/π) where A is the cross-sectional area. The needle may be provided with a "ribbon" shape with a single set of opposing flat sides, or a rectangular or "I-beam" shape, or with a cross-section which smoothly undergoes transition from the point to a circular cross-section, to a rectangular cross-section having rounded and then sharper corners, as described in U.S. Pat. No. 4,799,484.

The suture needle may be straight or curved. Preferably, the needle is curved through a radius of curvature, which need not be constant but is preferably constant. Thus, more preferred shapes of the needles of the present invention comprise sections of a circle, such as a quarter circle, three-eighths circle, half circle, or five-eighths of a circle.

Following the final drawing of the tungsten alloy wire to the final desired diameter, one end of the needle is given a point having the desired shape, the point being provided by any conventional technique such as grinding. Optionally, the body may be formed by pressing or grinding operations into the variety of shapes. The needle may then be given its desired curvature, typically by rolling around a mandrel of the desired radius of curvature. The opposite end of the needle is given an opening in its end, or other means by which the end of a suture can be attached to the needle by swaging or the like.

In order to impart improved bending strength and stiffness to the suture needle described herein, particularly after a curvature has been imparted to the needle, the curved needle is heated to a temperature below the recrystallization temperature of the tungsten alloy. It shall be noted that for purposes of this disclosure, recrystallization temperature is defined as any temperature in which the microstructure of the tungsten alloy suture needles may be changed via the formation of new grains. Preferably, the suture needle is heated to a temperature ranging from about 700 to about 1900 °C. In one embodiment of the invention, the suture needle is heated to a temperature ranging from about 800 to about 1150 °C in an inert or reducing atmosphere for about 0.5 hours to impart bending stiffness to the surgical needle. Needles may also be attached to a tape or other conveyer material and passed transiently in the vicinity of a heat source. In this way the exposure time to elevated temperature would be limited, since it will be recognized that higher temperatures for shorter periods of time are effective to achieve the desired stiffening effect. Examples of an inert or reducing atmosphere include, but are not limited to, vacuum, argon gas, nitrogen gas, hydrogen gas, or gas mixtures thereof.

The suture needles of the present invention are characterized by a desirable combination of bending stiffness, strength and ductility. For the needles of the present invention, the wire tensile yield strength is generally at least about 1723x10⁹ Pa (250,000 ksi). A high wire tensile yield strength is useful as it indicates the ability of the needles of the present invention to withstand potentially deforming stresses without suffering permanent deformation.

The wire from which the needles of the present invention are made also exhibits uniquely high Young's modulus of elasticity, generally at least about 400 GPa. The high Young's modulus is desirable in that it reflects the potential for higher stiffness and the ability of the needles of the present invention to withstand potentially deforming stresses by retaining their shape, without undue flexing. However, in practice, as described above, a high Young's modulus of the wire alone does not directly translate into a high bending stiffness for a curved suture needle. Indeed to capitalize on the intrinsic material stiffness, a heat treatment is applied to the curved suture needles, as described above.

An electrochemical process in an aqueous solution is employed to impart a robust adherent surface coloration to medical devices such as the tungsten alloy suture needles described herein. More particularly, suture needles may be connected to an anode, submerged in an aqueous solution having a pH of less than or equal to 7, and subjected to DC or AC potentials ranging from 1 to 40 VDC. Blue yellow and / or black surface colorations are produced in this way. These surface colors correspond to the colors exhibited by the various tungsten oxides.

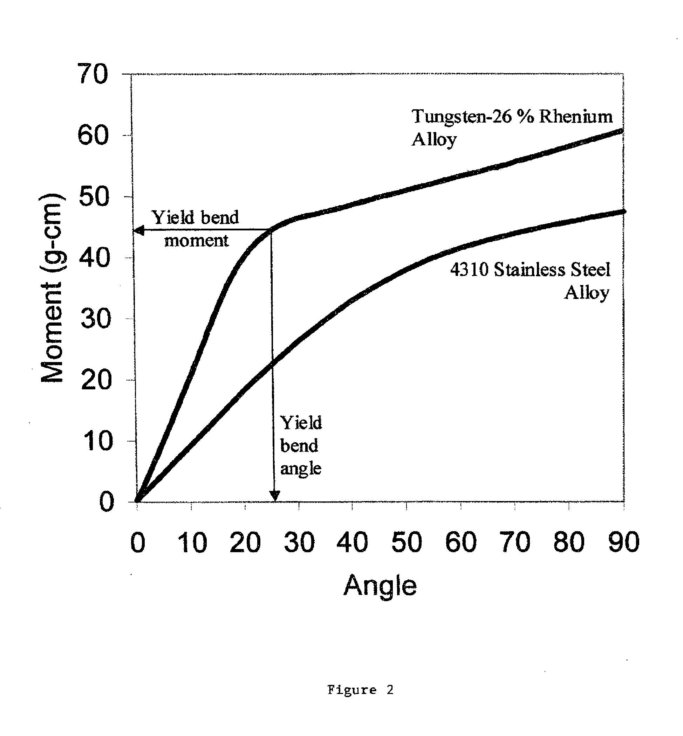

A transition in surface coloration from yellow to blue occurs at ∼ 20 VDC (pH<7, at room temperature). Below this potential, stoichiometric yellow oxide (WO₃) may form on the needle surface, whereas above this potential the non-stoichiometric blue oxide (WO_{<3}) may form. As indicated in Figure 2, the oxide stoichiometry and needle coloration depends upon the applied potential.

While tungsten has not been reported to exist as an ion in solution, the WO₄₋ tungstate ion commonly forms in alkaline solutions (Lillard et al.). In basic solutions, electropolishing the tungsten alloy suture needles was observed to advantageously remove minor amounts of surface materials for the purposes of cleaning the tungsten alloy suture needles or sharpening the needle points. Specifically, electropolishing and material removal was conducted by applying a potential across the needles in a basic solution. For example, sodium hydroxide mixed with water at concentration of about 1 to 25% NaOH by weight was found to be effective for electropolishing and material removal. Gycerol or other organic additives may be included in the solution to help control material removal throughout the electropolishing process. The solution may further be heated to a temperature above room temperature but below 100 °C to further modify the properties of these basic electropolishing solutions. As such, a limit may be placed on the solution pH that is effective for the coloration of the suture needle. Above a pH of 7, it has been discovered that surface oxides do not persist, but rather material is removed and the needles are made to have a "silver" appearance.

Finally, the needle may also be provided with a coating, for instance, a polymeric coating, in accordance with known techniques, if desired. The needle is then attached to the suture, packaged and sterilized, again in accordance with conventional techniques.

The properties of the suture needles of the present invention are illustrated in the following examples, which are provided for purposes of illustration and should not be interpreted as limiting in any way the scope of the claims appended hereto.

### Example 1

ASTM standard F1840-98a (Reapproved 2004) provides standard terminology for surgical suture needles and ASTM standard F1874-98 (Reapproved 2004) provides details of a standard test method for bend testing of needles used in surgical sutures. Both ASTM standards are incorporated herein by reference. Two different measures for the strength of surgical suture needles are used, namely, yield bend moment, which is the amount of moment required to initiate plastic deformation during a bend test, and maximum bend moment, which is the greatest moment applied to a needle during a bend test. This later value of maximum bend moment is typically measured at a point where the needle has undergone substantial plastic deformation and is generally higher than the yield bend moment or point at which plastic deformation initiates. The point of deflection at which plastic deformation initiates, or more formally according to ASTM standards, the angle at which the yield bend moment occurs, is referred to as the yield bend angle.

Both needle bending strength and needle bending stiffness influence handling characteristics, as well as penetration performance and efficacy of the suture needle. It is important to note that in almost all circumstances, the suture needle should be used in applications where the yield bend moment is not exceeded, since above this value, the needle will bend plastically, losing its original shape, and will no longer function as intended. It is thus apparent that a desirable characteristic of a suture needle is a high yield bend moment, which is a manifestation of the bending strength of the suture needle. Below the yield bend moment, the resistance of bending of the suture needle is best characterized by the needle bending stiffness. Needle bending stiffness is a critical measure of the resistance to elastic, or recoverable bending of the suture needle before needle deflection reaches the yield bend angle and can be calculated as the yield bend moment divided by the yield bend angle. If a straight or curved suture needle has a low value of bending stiffness, substantial bending of the needle will occur for a given bend moment, whereas if a straight or curved suture needle exhibits a high bending stiffness value, relatively little elastic bending of the needle will occur for a given bend moment. Surgeons will tend to perceive a high degree of elastic bending as a loss of control or as a poor penetration performance since the needle point is not translating directly with the motion of their hands. As such, needle bending stiffness may be recognized as a quintessential measure of needle performance in most surgical applications.

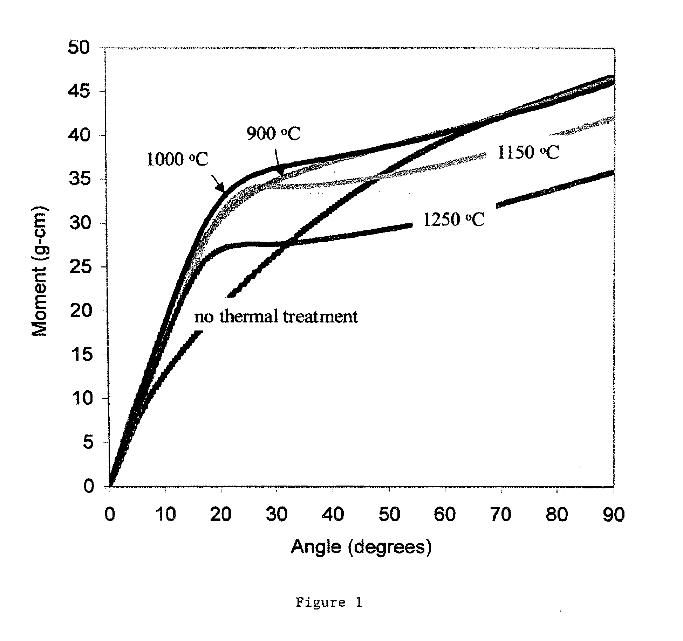

A graph comparing the bending performance of a curved 203 µm (.008") diameter suture needle produced from a tungsten 26% rhenium alloy to an equivalent curved suture needle produced from a commercial 4310 stainless steel alloy used in the manufacture of suture needles is provided in Figure 1. All tests were conducted according to ASTM standard F1874-98. The yield bend moment and yield bend angle are marked on the graph. The slope of the tungsten-rhenium alloy suture needle up to the yield bend moment represents bending stiffness and is markedly greater than that provided by the equivalent 4310 stainless steel alloy.

### Reference Example 2

A graph comparing the bending performance of curved 203 µm (.008") diameter suture needles produced from a tungsten 25.75% rhenium alloy after thermal treatment for 0.5 hr over a range of temperature is shown in Figure 2. Heat treatment was conducted under argon gas to maintain an inert non-oxidizing atmosphere. All tests were conducted according to ASTM standard F1874-98. A marked increase in bending stiffness occurs with the application of heat treatment. A maximum in bending stiffness is attained with a thermal treatment of 1000 °C for 0.5 hr. At temperatures above and below 1000 °C a decrease in the yield bend moment occurs.

It shall be recognized that similar results may be achieved with shorter duration thermal treatments at elevated temperatures and result in an upward shift for the optimal heat treatment temperature. Likewise, extended duration thermal treatments at lower temperatures may also be effective and result in a downward shift of the optimal treatment temperature.

### Example 3

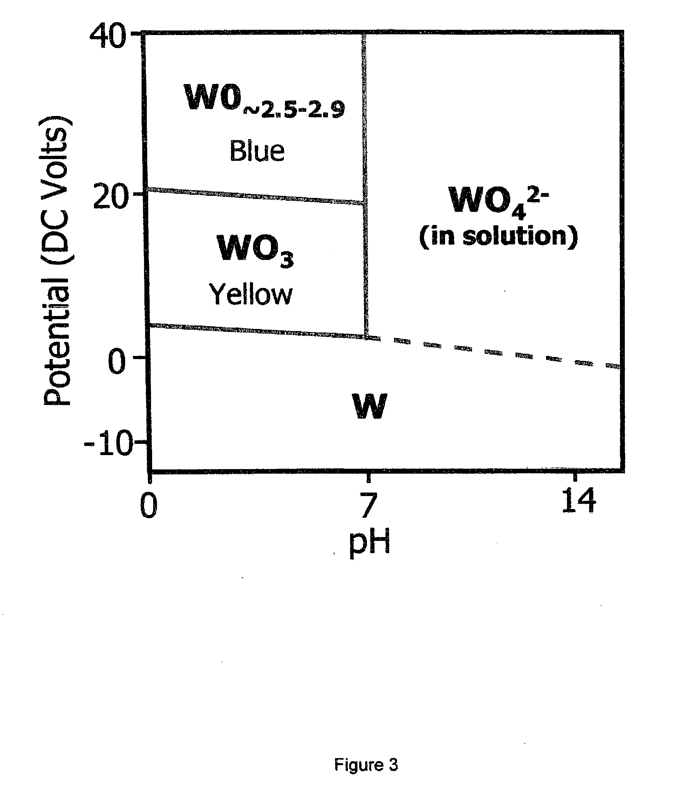

Tungsten and its alloys can form a yellow oxide with a stoichiometry of WO₃, and a blue oxide with a stoichiometry range of about WO_{2.5} to WO_{2.9}. The colors of the tungsten alloy needles observed in this study are attributable to the formation of a yellow or blue oxide on the needle surface. A process diagram that generalizes the effect of pH and direct potential on the formation of surface oxides is shown in Figure 3.

In this study, tungsten-25.75% rhenium needles were treated at room temperature in the various solutions shown in Table 1 below. The maximum pH for oxide formation was observed to be 7 in a 4% NaCl solution.

**TABLE 1***

| **Solution Type** | **Solution Concentration** | **Voltage** | **AC/DC (measurement)** | **Time (s)** | **Temperature (oC)** | **Color** |
|---|---|---|---|---|---|---|
| phosphoric acid | 70-85% | 5 | DC | 30 | 24C | silver/yellow |
| phosphoric acid | 70-85% | 10 | DC | 30 | 24C | yellow |
| phosphoric acid | 70-85% | 15 | DC | 30 | 24C | yellow |
| phosphoric acid | 70-85% | 20 | DC | 30 | 24C | yellow |
| phosphoric acid | 70-85% | 25 | DC | 30 | 24C | blue |
| phosphoric acid | 70-85% | 30 | DC | 30 | 24C | blue |
| phosphoric acid | 70-85% | 35 | DC | 30 | 24C | blue |
| phosphoric acid | 70-85% | 40 | DC | 30 | 24C | blue |
| | | | | | | |
| phosphoric acid | 50% | 15 | DC | 30 | 24C | yellow |
| phosphoric acid | 50% | 20 | DC | 30 | 24C | blue |
| phosphoric acid | 50% | 25 | DC | 30 | 24C | blue |
| | | | | | | |
| phosphoric acid | 25% | 5 | DC | 30 | 24C | yellow |
| phosphoric acid | 25% | 10 | DC | 30 | 24C | yellow |
| phosphoric acid | 25% | 15 | DC | 30 | 24C | yellow( |
| phosphoric acid | 25% | 20 | DC | 30 | 24C | blue |
| phosphoric acid | 25% | 25 | DC | 30 | 24C | blue |
| | | | | | | |
| phosphoric acid | 70-85% | 0.32 | AC | 30 | 24C | none |
| phosphoric acid | 70-85% | 0.7 | AC | 30 | 24C | Yellow/blue Tip |
| phosphoric acid | 70-85% | 1.1 | AC | 30 | 24C | Black/Blue |
| phosphoric acid | 70-85% | 1.39 | AC | 30 | 24C | black |
| phosphoric acid | 70-85% | 1.6 | AC | 30 | 24C | Blue/green |
| phosphoric acid | 70-85% | 2.2 | AC | 30 | 24C | green/yellow |
| phosphoric acid | 70-85% | 3.35 | AC | 30 | 24C | Black/blue |
| phosphoric acid | 70-85% | 4.9 | AC | 30 | 24C | black |
| | | | | | | |
| phosphoric acid | 75% | 1 | DC | 30 | 24C | silver |
| | | | | | | |
| nitric acid | 68-70% | 15 | DC | 30 | 24C | yellow |
| nitric acid | 68-70% | 20 | DC | 30 | 24C | blue/yellow |
| nitric acid | 68-70% | 22 | DC | 30 | 24C | blue |
| nitric acid | 68-70% | 25 | DC | 30 | 24C | blue |
| nitric acid | 68-70% | 25 | DC | 60 | 24C | blue |
| | | | | | | |
| hydrochloric acid | 37% | 15 | DC | 30 | 24C | yellow |
| | | | | | | |
| hydrochloric acid | 37% | 25 | DC | 30 | 24C | blue purple |
| | | | | | | |
| hydrochloric acid | 37% | 35 | DC | 30 | 24C | blue |
| | | | | | | |
| oxalic acid | 10% | 10 | DC | 30 | 24C | yellow |
| oxalic acid | 10% | 15 | DC | 30 | 24C | yellow |
| oxalic acid | 10% | 20 | DC | 30 | 24C | yellow |
| oxalic acid | 10% | 25 | DC | 30 | 24C | blue |
| | | | | | | |
| Sulfuric acid | 98% | 25 | DC | 30 | 24C | no effect |
| Sulfuric acid | 7% | 15 | DC | 30 | 24C | yellow (top), blue(bottom) |
| | | | | | | |
| Sulfuric acid | 7% | 25 | DC | 30 | 24C | blue |
| Sulfuric acid | 7% | 35 | DC | 30 | 24C | blue |
| | | | | | | |
| Acetic | pure | 25 | DC | 30 | 24C | no effect |
| Acetic | 10% | 15 | DC | 30 | 24C | yellow |
| Acetic | 10% | 25 | DC | 30 | 24C | blue |
| | | | | | | |
| Tap Water | 100% | 15 | DC | 30 | 24C | yellow |
| Tap Water | 100% | 25 | DC | 30 | 24C | blue |
| Tap Water | 100% | 35 | DC | 30 | 24C | blue |

| | | | | | | |
|---|---|---|---|---|---|---|
| *Needles processed individually, connected to anode, submerged - 1" in solution Colors reported where determined via visual inspection and may have been of dark or light hue. Furthermore, at the transition potential of - 20 VDC a best visual judgment was made as to colors that bordered between blue or yellow. The same colors may be interpreted as dark purple or black by different inspectors. | | | | | | |

A mechanism for the formation of the surface oxide from an aqueous solution is proposed below.

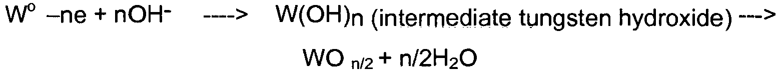

The fact that the coloration process is effective in a wide variety of aqueous solutions including acids that do not contain oxygen in their chemical make up, e.g. hydrochloric acid (HCl) and salt (NaCl) water, lends support to this simple mechanism for surface oxide formation.

It should also be noted that the oxides/hydroxides that form on the surface of the needle are insulative, and once formed, the current passage or potential will decrease. As a further point, reheating the needles either in an inert or oxidative atmosphere, may improve the oxide surface coloration or modify color tones upon transformation of hydroxides to oxides while releasing water.

## Claims

1. A method for making a suture needle comprising the steps of (1) forming needle blanks of a tungsten alloy comprising up to 30 weight percent of one or more metals selected from rhenium, osmium, tantalum and molybdenum, and the balance tungsten, into a suture needle; and (2) subjecting the suture needle to a DC or AC potential ranging from 1 to 40 VDC while submerged in an aqueous solution with a pH of 7 or less, to impart a yellow, blue or black surface coloration to the needle.

2. The method of claim 1, wherein the potential is greater than 20 volts and the surface coloration is blue.

3. The method of claim 1, wherein the potential is less than 20 volts and the surface coloration is yellow.

4. The method of claim 1, wherein the potential is about 20 volts and the surface coloration is black.

5. The method of claim 1, where the solution comprises one or more of the group consisting of hydrochloric acid, phosphoric acid, sulfuric acid, nitric acid, oxalic acid, acetic acid, water, or salt water.

6. The method according to any preceding claim, wherein the tungsten alloy comprises rhenium.

7. The method according to any preceding claim, wherein the tungsten alloy comprises 20 to 26 weight percent rhenium, and the balance tungsten.

8. A suture needle obtainable by a method according to any of claims 1 to 7.

9. The suture needle of claim 8, where the suture needle is curved.

## Patentansprüche

1. Verfahren zum Herstellen einer Nähnadel, umfassend die Schritte (1) Formen von Nadelrohlingen aus einer Wolframlegierung, die bis zu 30 Gewichtsprozent an einem oder mehreren Metallen ausgewählt aus Rhenium, Osmium, Tantal und Molybdän und als Rest Wolfram umfasst, zu einer Nähnadel; und (2) Unterwerfen der Nähnadel eingetaucht in eine wässrige Lösung mit einem pH-Wert von 7 oder weniger an ein DC- oder AC-Potential im Bereich von 1 bis 40 VDC, um der Nadel eine gelbe, blaue oder schwarze Oberflächenfärbung zu verleihen.

2. Verfahren gemäß Anspruch 1, wobei das Potential höher als 20 Volt ist und die Oberflächenfärbung blau ist.

3. Verfahren gemäß Anspruch 1, wobei das Potential niedriger als 20 Volt ist und die Oberflächenfärbung gelb ist.

4. Verfahren gemäß Anspruch 1, wobei das Potential etwa 20 Volt beträgt und die Oberflächenfärbung schwarz ist.

5. Verfahren gemäß Anspruch 1, wobei die Lösung eines oder mehrere aus der Gruppe bestehend aus Salzsäure, Phosphorsäure, Schwefelsäure, Salpetersäure, Oxalsäure, Essigsäure, Wasser und Salzwasser umfasst.

6. Verfahren gemäß einem der vorstehenden Ansprüche, wobei die Wolframlegierung Rhenium umfasst.

7. Verfahren gemäß einem der vorstehenden Ansprüche, wobei die Wolframlegierung 20 bis 26 Gewichtsprozent Rhenium und als Rest Wolfram umfasst.

8. Nähnadel, erhältlich durch ein Verfahren gemäß einem der Ansprüche 1 bis 7.

9. Nähnadel gemäß Anspruch 8, wobei die Nähnadel gekrümmt ist.

## Revendications

1. Procédé de fabrication d'une aiguille pour suture, le procédé comportant les étapes qui consistent à
(1) former des ébauches d'aiguille en alliage de tungstène comprenant jusque 30 pour cent en poids d'un ou plusieurs métaux sélectionnés parmi le rhénium, l'osmium, le tantale et le molybdène, le solde étant le tungstène, pour obtenir une aiguille de suture et
(2) appliquer sur l'aiguille de suture un potentiel en courant continu ou en courant alternatif compris entre 1 et 40 V en courant continu en l'immergeant dans une solution aqueuse dont le pH est de 7 ou moins, pour donner une coloration jaune, bleue ou noire à la surface de l'aiguille.

2. Procédé selon la revendication 1, dans lequel le potentiel est supérieur à 20 volts et la coloration de la surface est le bleu.

3. Procédé selon la revendication 1, dans lequel le potentiel est inférieur à 20 vols la coloration de surface est le jaune.

4. Procédé selon la revendication 1, dans lequel le potentiel est d'environ 20 volts et la coloration de surface est le noir.

5. Procédé selon la revendication 1, dans lequel la solution contient un ou plusieurs des éléments de l'ensemble constitué de l'acide chlorhydrique, de l'acide phosphorique, de l'acide sulfurique, de l'acide nitrique, de l'acide oxalique, de l'acide acétique, de l'eau et de l'eau salée.

6. Procédé selon l'une quelconque des revendications précédentes, dans lequel l'alliage de tungstène contient du rhénium.

7. Procédé selon l'une quelconque des revendications précédentes, dans lequel l'alliage de tungstène contient de 20 à 26 pour cent en poids de rhénium, le solde étant le tungstène.

8. Aiguille de suture obtenue par le procédé selon l'une des revendications 1 à 7.

9. Aiguille de suture selon la revendication 8, l'aiguille de suture étant incurvée.
